# EUROPEAN PATENT APPLICATION

(11) **EP 2 774 643 A1**
(43) Date of publication of application: **10.09.2014**
(21) Application number: 12846777.6
(22) Date of filing: 02.11.2012
(51) Int. Cl.: A61M 5/32, A61M 5/158

(54) **INJECTION NEEDLE**

(30) Priority: 04.11.2011 JP 2011242294
(71) Applicant: Nipro Corporation, Osaka-shi, Osaka 531-8510 (JP)
(72) Inventor: IWASE, Yoshiharu, Tatebayashi-shi Gunma 374-8518 (JP); EIZUMI, Yutaka, Tatebayashi-shi Gunma 374-8518 (JP); SASAKI, Isao, Tatebayashi-shi Gunma 374-8518 (JP)
(74) Representative: Oxley, Rachel Louise
(86) International application number: PCT/JP2012/078443
(87) International publication number: WO 2013/065814

(57) **Abstract**

An injection needle 1 made of an alloy containing cobalt and molybdenum is manufactured as follows. First, lancet cut is carried out to a pipe-like metallic material composed of the alloy containing cobalt and molybdenum to form a blade tip 3 and a blade part 5. Then, a distal end part 1A including the blade tip 3 is mechanically polished, and thereafter, the distal end part 1A is immersed in an electrolytic solution and electrolytically polished. Thus, burrs at the blade tip 3 or the like generated during mechanical polishing are removed. Since the injection needle 1 is composed of the alloy containing cobalt and molybdenum, compared to a conventional injection needle made of stainless steel, an injection needle in which surface roughness of an inner peripheral surface of the injection needle is low and smooth can be provided.

## Description

### Technical Field

The present invention relates to an injection needle, and, more specifically relates to an injection needle composed of an alloy containing cobalt and molybdenum.

### Background Art

Conventionally, an improvement plan for reducing implement resistance and channel resistance in a metallic injection needle is proposed (for instance, Patent Literature 1). That is, the Patent Literature 1 discloses an injection needle including a tapered part between an impalement part to be a blade tip and a large-diameter base part.

### Prior Art Document

### Patent Literature

Patent Literature 1: Japanese Patent Laid-Open No.2008-200528

### Summary of Invention

### Problems to be Solved by the Invention

Since conventional injection needles including the one in Patent Literature 1 are made of stainless steel, there is a problem that a surface of an inner peripheral surface of the injection needle becomes rough and channel resistance increases because of that. Also, in the injection needle made of stainless steel, when an outer diameter thereof is to be reduced, a distal end part tends to be broken or damaged so that there is a limit in reduction of the outer diameter for reducing implement resistance.

### Means for Solving the Problems

In consideration of the above-described circumstances, in the present invention, in an injection needle made of a metal with a blade tip and a blade part formed at a distal end part, the metal is an alloy containing cobalt and molybdenum.

### Advantageous Effects of Invention

Such a configuration can provide an injection needle for which surface roughness of an inner peripheral surface of the injection needle is low and smooth compared to conventional products, as is clear from results of tests and measurement described later.

### Brief Description of Drawings

[Figure 1] Figure 1 is a plan view of a main section illustrating one embodiment of the present invention.
[Figure 2] Figure 2 is a side view from a direction of an arrow II in Figure 1.
[Figure 3] Figure 3 is a manufacturing process diagram of an injection needle of the present embodiment illustrated in Figure 1, Figure 3(a) illustrates a process of molding a metallic material, Figure 3(b) illustrates a first process of lancet cut, and Figure 3(c) illustrates a second process of the lancet cut.
[Figure 4] Figure 4 is a diagram illustrating compositions of elements of the injection needle of the present embodiment illustrated in Figure 1 and a conventional product.
[Figure 5] Figure 5 is a diagram illustrating dimensions of respective parts of the injection needle of the present embodiment illustrated in Figure 1 and a conventional product.
[Figure 6] Figure 6 is a diagram illustrating a test result of a surface roughness test conducted to the injection needle of the present embodiment illustrated in Figure 1 and a conventional product.
[Figure 7] Figure 7 is a diagram illustrating a test result of a liquid flow rate test conducted to the injection needle of the present embodiment illustrated in Figure 1 and a conventional product.
[Figure 8] Figure 8 is a diagram illustrating a test result of a hardness/rigidity test conducted to the injection needle of the present embodiment illustrated in Figure 1 and a conventional product.
[Figure 9] Figure 9 is an enlarged photo (backscattered electron image of ×150 magnification) of a distal end part in the injection needle of the present embodiment illustrated in Figure 1, Figure 9(a) illustrates the most distal end part (blade tip), Figure 9(b) illustrates a center part, and Figure 9(c) illustrates a rearmost part.
[Figure 10] Figure 10 is an enlarged photo (backscattered electron image of ×150 magnification) of a distal end part in the injection needle which is a conventional product, Figure 10(a) illustrates the most distal end part (blade tip), Figure 10(b) illustrates a center part, and Figure 12(c) illustrates a rearmost part.
[Figure 11] Figure 11 is an enlarged photo (backscattered electron image of ×500 magnification) of a distal end part in the injection needle of the present embodiment illustrated in Figure 1, Figure 11(a) illustrates the most distal end part (blade tip), Figure 11(b) illustrates a center part, and Figure 13(c) illustrates a rearmost part.
[Figure 12] Figure 12 is an enlarged photo (backscattered electron image of ×500 magnification) of a distal end part in the injection needle which is a conventional product, Figure 12(a) illustrates the most distal end part (blade tip), Figure 12(b) illustrates a center part, and Figure 12(c) illustrates a rearmost part.
[Figure 13] Figure 13 is an enlarged photo (backscattered electron image of ×1000 magnification) of an inner peripheral surface in the injection needle of the present embodiment illustrated in Figure 1.
[Figure 14] Figure 14 is an enlarged photo (backscattered electron image of ×1000 magnification) of an inner peripheral surface in the injection needle which is a conventional product.

### Mode for Carrying out the Invention

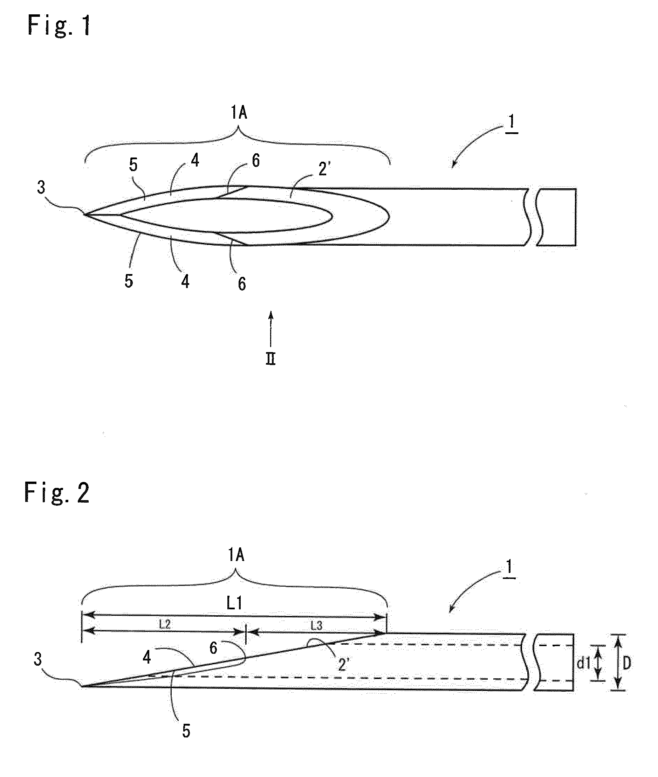

Hereinafter, describing the present invention for an illustrated embodiment, in Figure 1 and Figure 2, reference numeral 1 denotes an injection needle made of a metal, and a blade tip 3 and a blade part 5 and the like are formed at a distal end part 1A of the injection needle 1 through a manufacturing process of lancet cut or the like similarly to a conventional product. While a composition of a material will be described later in detail, the injection needle 1 of the present embodiment is characterized in that an alloy containing cobalt and molybdenum is used as the material (metallic material).

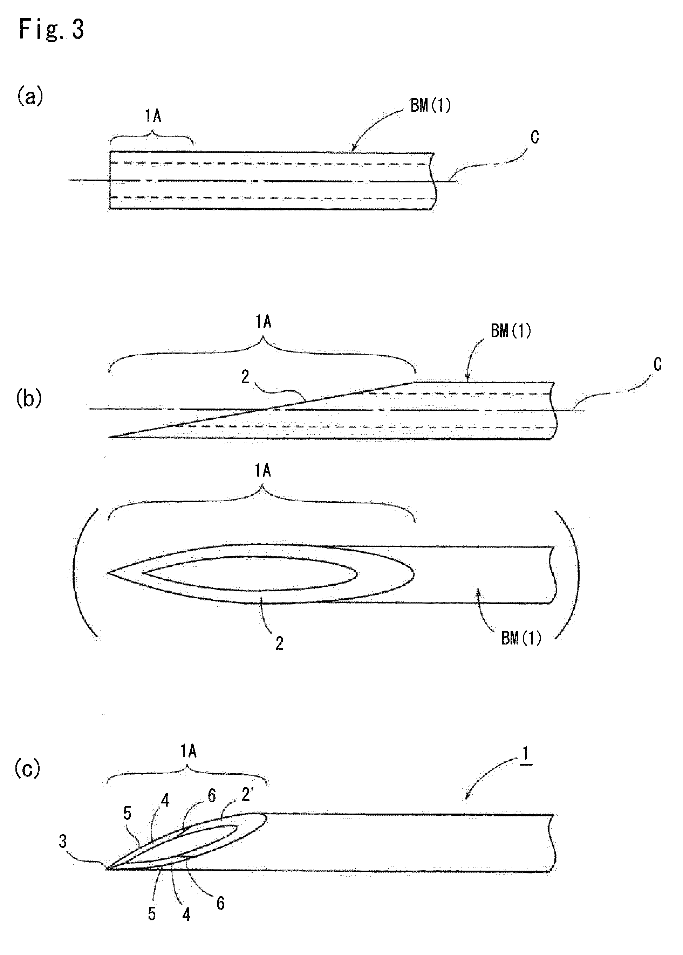

Here, the manufacturing process of the injection needle 1 of the present embodiment will be described with Figure 3. That is, first, a thin plate composed of the alloy containing cobalt and molybdenum is molded into a cylindrical shape and a metallic material BM in a thin and long cylindrical shape is produced (see Figure 3(a)). Then, the distal end part 1A of the metallic material BM is cut obliquely at a required angle to an axial center C (see Figure 3(a)-Figure 3(b)). Thus, a loop-like primary inclined surface 2 is formed at the distal end part 1A (see Figure 3(b)). Thereafter, the metallic material BM is rotated reciprocally by a required angle with the axial center C as a rotation center and then both left and right sides of a tip side of the primary inclined surface 2 are obliquely cut further (see Figure 3(c)).

Thus, a tip part to be the blade tip 3 is formed, secondary inclined surfaces 4, 4 are formed on the left and right of an adjacent rear part thereof, and a remaining primary inclined surface 2' is formed further at an adjacent rear part thereof. Also, edge parts at the outer part of the left and right secondary inclined surfaces 4, 4 and the remaining primary inclined surface 2' are formed as the blade parts 5, 5. Also, at a boundary part of the secondary inclined surfaces 4, 4 and the remaining primary inclined surface 2', linear swollen parts 6, 6 due to inclination angles in the two cutting processes are generated.

Thus, a shape of the distal end part 1A of the injection needle 1 is roughly completed, and the left and right secondary inclined surfaces 4, 4 and the remaining primary inclined surface 2' are mechanically polished thereafter. The blade tip 3 and the blade parts 5, 5 are polished by the mechanical polishing, however, fine burrs from the mechanical polishing are generated at the parts (not shown in the figure). Then, as a final process thereafter, the entire distal end part 1A including the blade tip 3 and the blade part 5 is immersed in an electrolytic solution and the distal end part 1A is electrolytically polished. Here, the injection needle 1 of the present embodiment composed of the alloy containing cobalt and molybdenum has a problem that, when the time of electrolytic polishing is too long, the blade tip 3 becomes round even though the burrs at the blade parts 5, 5 are removed. Then, in the present embodiment, as the time of electrolytically polishing the distal end part 1A, the time for removing the burrs from the blade tip 3 and the blade parts 5, 5 and achieving smooth finish is set. Thus, the fine burrs generated during the mechanical polishing in the previous process are removed from the blade tip 3 and the blade part 5 and final polishing is carried out to the parts.

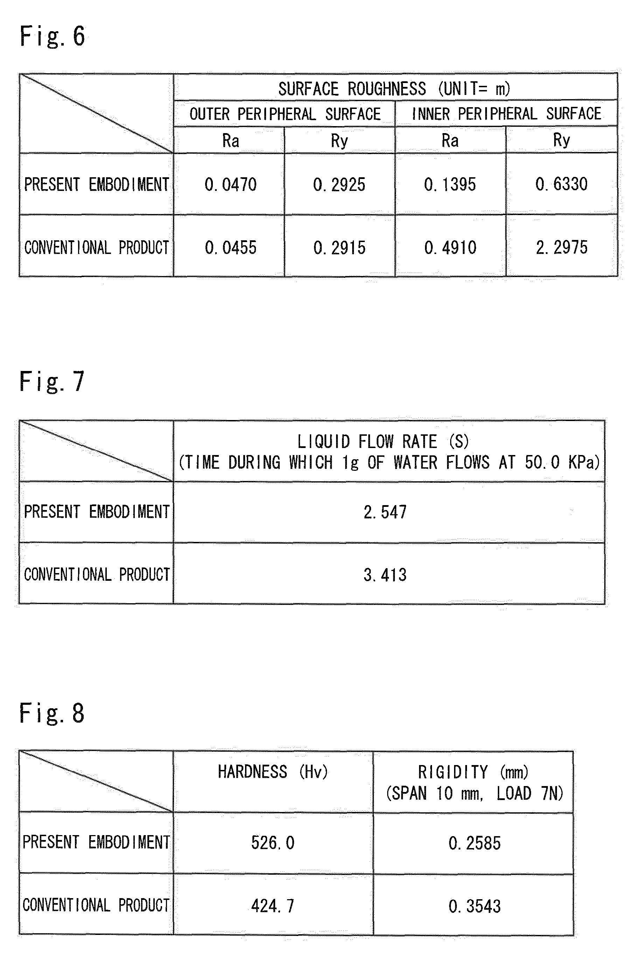

The blade tip 3 and the blade part 5 are formed at the distal end part 1A in such a manner, and the manufacturing process of the injection needle 1 of the present embodiment is ended. A method of cutting the distal end part of the material BM illustrated in Figure 3(b)-Figure 3(c) is conventionally and generally called "lancet cut". The above-described manufacturing process of the injection needle 1 of the present embodiment itself is the same as the conventional well-known manufacturing process, and a configuration of the distal end part 1A to which the lancet cut is carried out is also well-known (see Figure 7 in Patent Literature 1).

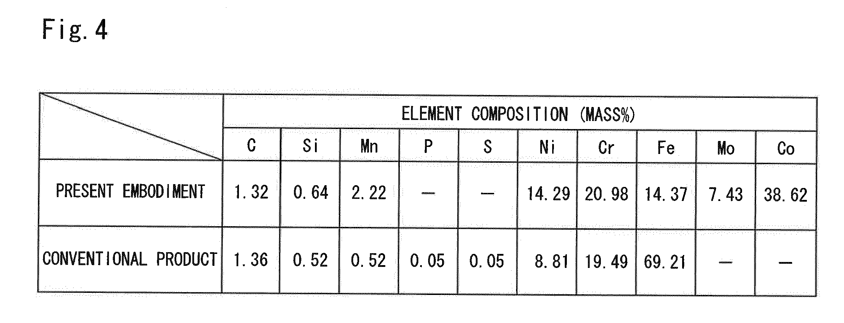

As described above, the injection needle 1 of the present embodiment is composed of the alloy containing cobalt and molybdenum, and the composition of the alloy is illustrated in an upper stage of Figure 4. That is, the composition of the injection needle 1 of the present embodiment is constituted of the alloy formed of 38.62 mass% cobalt, 20.987 mass% chrome, 14.37 mass% iron, 14.29 mass% nickel, 7.43 mass% molybdenum, and the balance being the other elements. In other words, the material of the injection needle 1 is the alloy containing cobalt and molybdenum, with cobalt being a main component. A composition ratio of the respective elements is, preferably, 39.00-42.00 mass% cobalt, 18.00-21.50 mass% chrome, 14.00-18.00 mass% nickel, and 6.50-8.00 mass% molybdenum. For numerical values of the composition ratio of the respective elements described in Figure 4, average values of measured values at two or more parts in the injection needle 1 are described.

In the meantime, a lower stage of Figure 4 illustrates a composition of the injection needle 1 made of stainless steel, that is manufactured from a thin plate composed of an alloy of a conventional composition through the manufacturing process completely same as the present embodiment. That is, the composition of the conventional injection needle 1 is constituted of the alloy formed of 69.2 mass% iron, 19.49 mass% chrome, 8.81 mass% nickel, and the balance being the other elements. In other words, the material of the conventional injection needle 1 is the stainless steel with iron as a main component. For numerical values of the composition ratio of the respective elements described in Figure 4, an average value of measured values at two or more parts in the injection needle 1 which is a conventional product is described.

When an inventor of the present application carried out various kinds of tests and measurements for required items regarding the injection needle 1 of the present embodiment and the injection needle 1 of the conventional product, that are formed of the compositions illustrated in Figure 4, it was clarified that channel resistance is smaller in the injection needle 1 of the present embodiment, that is formed of the alloy containing cobalt and molybdenum, than in the conventional injection needle 1 made of stainless steel, or the like.

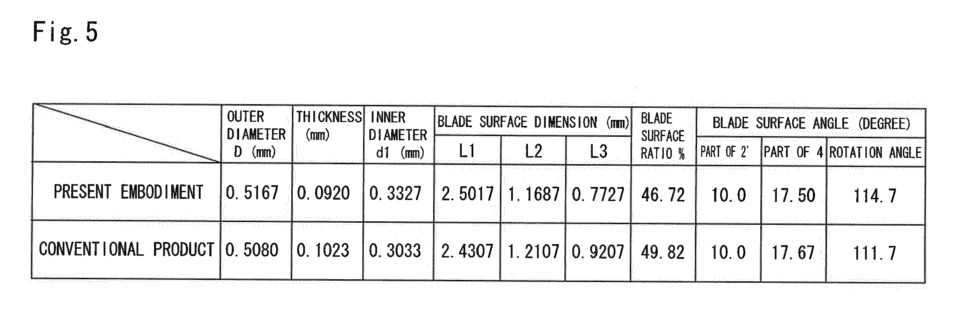

In order to test the injection needle 1 of the present embodiment and the conventional product, the inventor of the present application prepared three each of samples having the same outer diameter D and the same total length as test pieces, and conducted various kinds of tests to three each of the injection needles 1 of the present embodiment and the conventional product. Specific dimensions of respective parts are illustrated in Figure 5. Also, as illustrated in Figure 2, reference character D denotes an outer diameter, and reference character d1 denotes an inner diameter. Reference character L1 denotes an axial direction dimension of the distal end part 1A, reference character L2 denotes a shortest dimension from the blade tip 3 to the swollen part 6, and reference character L3 denotes an axial direction dimension from the swollen part 6 to the rearmost part of the distal end part 1A. Further, a blade surface angle indicates the respective inclination angles in the first process and the second process in the above-described lancet cut, and a rotation angle indicates the rotation angle of rotating the metallic material in the second process.

First, Figure 6 illustrates a measurement result regarding surface roughness of the injection needle 1 of the present embodiment and the conventional product. The measurement result indicates the average value of the measured values measured for three each of the samples of the injection needle 1 of the present embodiment and the conventional product. For the surface roughness of an outer peripheral surface of the injection needle 1, there is not a big difference between the injection needle 1 of the present embodiment and the conventional product. However, for the surface roughness of an inner peripheral surface, a numerical value of the injection needle 1 of the present embodiment is clearly smaller than that of the conventional product, and it is understood that the inner peripheral surface of the present embodiment is smoother than that of the conventional product.

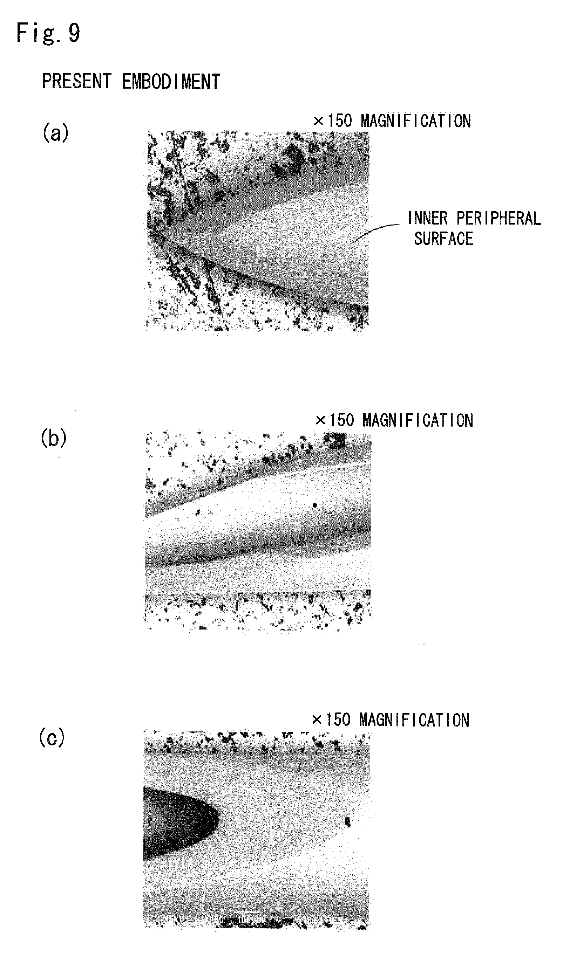

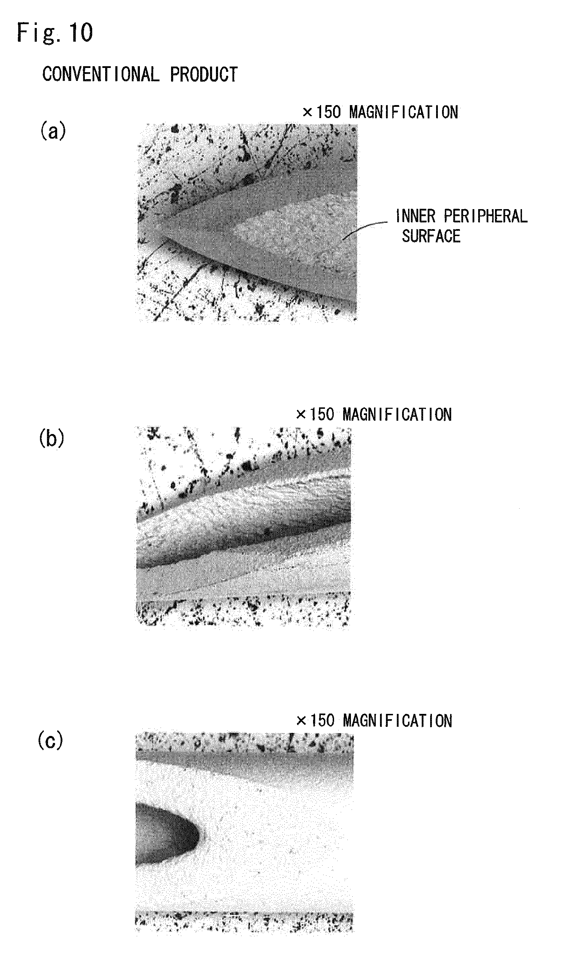

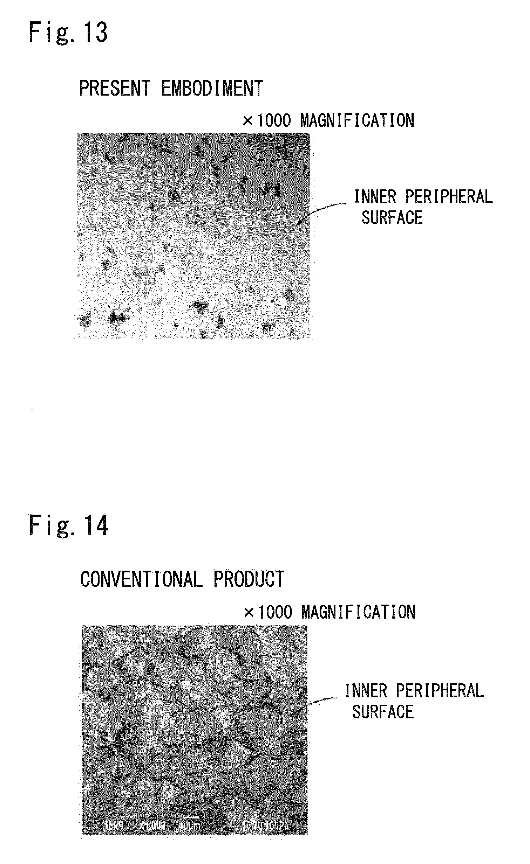

That fact is clear from enlarged photos comparing the distal end part and the inner peripheral surface of the injection needle 1, that are illustrated in Figure 9-Figure 14. While fine wrinkles are seen only partially on the inner peripheral surface of the injection needle 1 of the present embodiment as illustrated in Figure 9(a), Figure 11(a) and Figure 13, fine wrinkles are seen over the entire inner peripheral surface of the injection needle 1 of the conventional product as illustrated in Figure 10(a), Figure 12(a) and Figure 14.

It is considered that difference in roughness is generated between the inner peripheral surface of the injection needle 1 of the present embodiment and the inner peripheral surface of the conventional product in such a manner due to difference in the material of the injection needle 1. That is, since the injection needle 1 of the present embodiment is the alloy containing cobalt and molybdenum, wrinkles are hardly generated on the inner peripheral surface when the metallic material BM in a thin plate shape is molded into the thin and long cylindrical shape in the above-described manufacturing process. On the other hand, in the conventional product made of stainless steel, wrinkles are easily generated on the entire inner peripheral surface when stainless steel in the thin plate shape is molded into the cylindrical shape. Therefore, in comparison as finished products after being manufactured, roughness of the inner peripheral surface is lower and smoother in the injection needle 1 of the present embodiment than in the conventional product.

The difference in the roughness of the inner peripheral surface also appears as difference in a liquid flow rate of liquid distributed on an inner surface of the injection needle 1. That is, the time during which water is distributed inside the injection needle 1 is measured in Figure 7. In this experiment, the time during which 1g of water flows inside the injection needle 1 at 50.0 kPa is indicated. Figure 7 also illustrates the average value of results of conducting the test for three each of the injection needle 1 of the present embodiment and the conventional product. As illustrated in Figure 7, while it is 2.547 seconds for the injection needle 1 of the present embodiment, it is 3.413 seconds for the conventional product, and it is clear that the liquid flow rate of the present embodiment is higher than that of the conventional product. In other words, it is clear that the channel resistance is smaller in the present embodiment than in the conventional product. It is considered to be due to the difference in the roughness of the inner peripheral surface described above. In such a manner, according to the present embodiment, the injection needle 1 having the channel resistance smaller than that of the conventional product even with the same outer diameter as the conventional product can be provided.

Next, Figure 8 illustrates a result of measuring difference in hardness and rigidity regarding the injection needle 1 of the present embodiment and the conventional product. Figure 8 also illustrates the average value of measurement results for three each of the injection needle 1 of the present embodiment and the conventional product.

For the hardness, while it is 526.0 (Hv) in the present embodiment, it is 424.7 (Hv) in the conventional product. Clearly, the hardness is higher in the injection needle 1 of the present embodiment than in the conventional product.

The rigidity is measured as follows. That is, a deflection amount of the blade tip 3 when a load is applied to a part 10 mm behind the blade tip 3 is measured. While the deflection amount is 0.2586 mm in the present embodiment, it is 0.3548 mm in the conventional product. That is, it is clear that the rigidity is higher in the present embodiment than in the conventional product.

In this way, since the hardness and the rigidity are higher in the injection needle 1 of the present embodiment composed of the alloy containing cobalt and molybdenum than in the conventional product, the injection needle which is harder to break than the conventional product can be achieved. Therefore, the present invention can provide the injection needle 1 for which easiness of breaking that is a conventional problem in the injection needle with a small diameter is improved.

Also, since the injection needle 1 of the present embodiment is excellent in the hardness and the rigidity, the outer diameter of the injection needle 1 that is limited by an ISO standard can be extremely reduced. Therefore, the injection needle 1 of the present embodiment can reduce a generation rate of ISO nonconforming products, that rises accompanying the extreme reduction of the outer diameter. Also, since the outer diameter is small, the probability that the injection needle 1 is brought into contact with a pain acceptor when an injection is given to a patient can be reduced.

Further, since the injection needle 1 of the present embodiment can be thinner than before (see Figure 5, for instance), even when the outer diameter is the same as the conventional product, the injection needle 1 having the inner diameter larger than the conventional product can be manufactured. In this way, since the injection needle 1 with the large inner diameter can be provided, a flow rate of a liquid chemical distributed inside the injection needle 1 is increased and the injection needle 1 is not easily clogged with the liquid chemical. Also, inner pressure increase when administering the liquid chemical can be suppressed. Therefore, the injection needle 1 of the present embodiment is suitable when administering a hormone drug that tends to be crystallized by a pressure.

Also, use of the technology of the present invention can achieve an ultrafine diameter (the outer diameter being 0.185 mm to 0.200 mm, the thickness being 0.035 mm to 0.037 mm, and the inner diameter being 0.115 mm to 0.126 mm) that is difficult to be implemented by a conventional method using SUS.

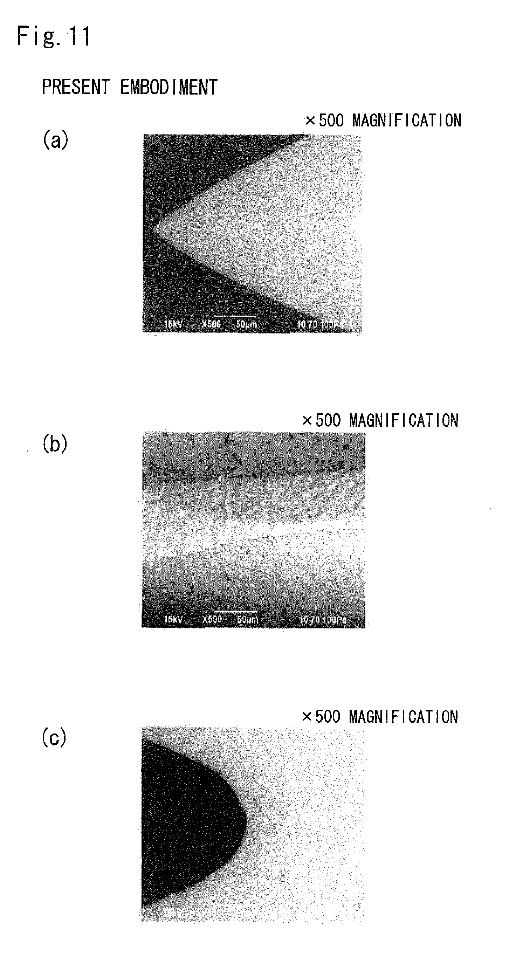

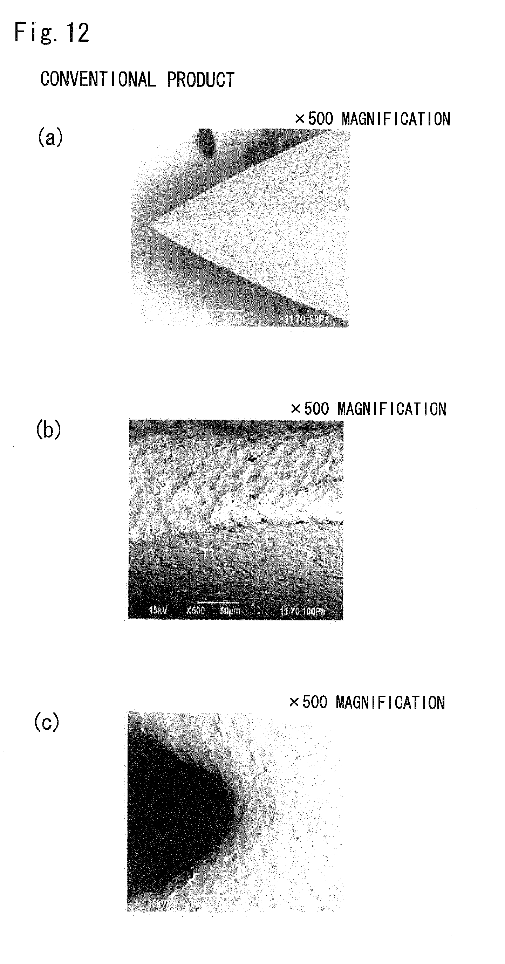

Further, for the injection needle 1 of the present embodiment, the burrs at the blade tip 3 and the blade part 5 are completely removed after the electrolytic polishing that is the last process, and the secondary inclined surfaces 4, 4 and the remaining primary inclined surface 2' are also finished to be smooth surfaces. Also, the swollen parts 6, 6 are rounded and a cross section thereof becomes a smooth circular arc shape. That is, it is clear when the present embodiment and the conventional product are compared by the enlarged photos in Figure 9-Figure 12. That is, as illustrated in Figure 9(a)-Figure 9(b) and Figure 11(a)-Figure 11(c), in the present embodiment, the blade tip 3 is turned to a state of being pointed at an acute angle, and the blade part 5 and the secondary inclined surfaces 4, 4 are finally polished to be smooth. The swollen parts 6, 6 to be the boundary part of the secondary inclined surfaces 4, 4 and the remaining primary inclined surface 2' are also rounded and turned to a smooth shape. On the other hand, as illustrated in Figure 10(a)-Figure 10(b) and Figure 12(a)-Figure 12(c), in the conventional product, though the blade tip 3 is turned to the acute angle, fine recesses and projections (burrs) remain at the blade parts 5, 5 and the secondary inclined surfaces 4, 4. Also, the swollen parts 6, 6 to be the boundary part of the secondary inclined surfaces 4, 4 and the remaining primary inclined surface 2' remain in an angular linear shape.

As described above, since the present embodiment is the injection needle 1 composed of the alloy containing cobalt and molybdenum, the distal end part 1A including the blade tip 3 and the blade parts 5, 5 is extremely smoothly finished after the electrolytic polishing. Then, in the electrolytic polishing, since electric resistance is small because the injection needle 1 contains cobalt, processing time of the electrolytic polishing can be made shorter than before.

In this way, since the blade tip 3, the blade part 5 and the swollen parts 6, 6 are smoothly finished in the injection needle 1 of the present embodiment, it is possible to reduce puncture resistance when puncturing the distal end part 1A of the injection needle 1 into a patient. Therefore, the present embodiment can reduce pains when puncturing the injection needle 1 into a patient compared to the conventional product.

While the embodiment describes the case of applying the present invention to the injection needle 1 to which the lancet cut is carried out, it is needless to say that the present invention is applicable to the other metallic injection needles to which the lancet cut is not carried out.

### Reference Signs List

- 1: Injection needle
- 1A: Distal end part
- 3: Blade tip
- 5, 5: Blade part

## Claims

1. An injection needle made of a metal with a blade tip and a blade part formed at a distal end part, wherein
the metal is an alloy containing cobalt and molybdenum.

2. The injection needle according to claim 1, wherein the alloy contains 39.00-42.00 mass% cobalt and 6.50-8.00 mass% molybdenum.

3. The injection needle according to claim 1 or claim 2, wherein the alloy contains chrome, iron, and nickel.

4. The injection needle according to claim 1, wherein the alloy contains 39.00-42.00 mass% cobalt, 6.50-8.00 mass% molybdenum, 18.00-21.50 mass% chrome, 14.00-18.00 mass% nickel, and 6.50-8.00 mass% molybdenum.

5. The injection needle according to any one of claims 1 to 4, wherein the blade tip and the blade part at the distal end part are finally polished to be smooth by electrolytic polishing.
